(19) Europäisches Patentamt
European Patent Office
Office européen des brevets

(11) Numéro de publication: **0 206 913 B1**

# (12) FASCICULE DE BREVET EUROPEEN

(45) Date de publication du fascicule du brevet: **13.09.89**

(51) Int. Cl.⁴: **C07H 21/00**

(21) Numéro de dépôt: **86401289.3**

(22) Date de dépôt: **13.06.86**

(54) **Nouveaux supports, la préparation de ces supports, les intermédiaires nouveaux obtenus, leur application à la synthèse d'oligonucléotides et les nouveaux nécléosides et oligonucléotides reliés aux supports ainsi obtenus.**

(30) Priorité: **20.06.85 FR 8509382**

(43) Date de publication de la demande: **30.12.86 Bulletin 86/52**

(45) Mention de la délivrance du brevet: **13.09.89 Bulletin 89/37**

(84) Etats contractants désignés: **BE CH DE FR GB IT LI LU NL SE**

(56) Documents cités:
**ZEITSCHRIFT FÜR CHEMIE, vol. 23, no. 9, 1983, pages 317-327, Leipzig, DD; A. ROSENTHAL et al.: "Chemische Synthese von DNA-Sequenzen"**
**Affinity Chromatography, J. Turkova, pages 180-181 (Elsevier, 1978)**

(73) Titulaire: **ROUSSEL-UCLAF, 35, boulevard des Invalides, F-75007 Paris(FR)**

(72) Inventeur: **Buendia, Jean, 3bis, Impasse Emilie, F-94170-Le Perreux Sur Marne(FR)**
Inventeur: **Nierat, Jeanine, 44, rue Carnot, F-92150-Suresnes(FR)**

(74) Mandataire: **Bourgouin, André et al, Département des Brevets ROUSSEL UCLAF B.P. no 9, F-93230 Romainville(FR)**

**EP 0 206 913 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

L'invention concerne l'application à la synthèse d'oligonucléotides de supports et les nouveaux nucléosides et oligonucléotides reliés aux supports ainsi obtenus.

De nombreux supports ont déjà été décrits dans la littérature pour la synthèse en phase solide d'oligonucléotides.

Ces supports sont par exemple uniquement constitués de polymères, tels que le polystyrène (Nucleic. Ac. Res. 1980, volume 8), le polyacrylamide acryloylmorpholide, le polydimethylacrylamide polymérisé sur kieselguhr [Nucleic Ac. Res. 9 (7) 1691 (1981)] de formule kieselguhr polyacrylamide

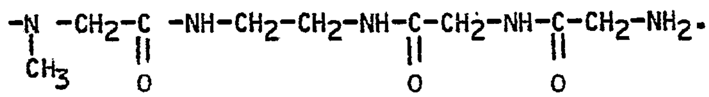

Ces supports se sont révélés insuffisants, car ils ont tendance à gonfler excessivement et à retenir certains réactifs.

D'autres supports déjà décrits sont de nature inorganique. On peut citer, par exemple, le support:

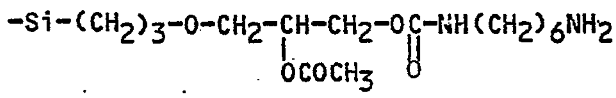

[J. Am. Chem. Soc., 105, 661 (1983)] ou le support à base de silice fonctionnalisée par un groupement 3-aminopropyltriéthoxysilane, dont l'utilisation en synthèse phosphite et phosphoramidite pour la préparation d'oligo-nucléotides a été décrite pour la première fois dans le brevet européen nº 0 035 719.

Cependant, ce support utilisé en synthèse phosphotriester donne de mauvais rendements, notamment lors des premiers couplages.

Un autre support à base de silice, de formule:

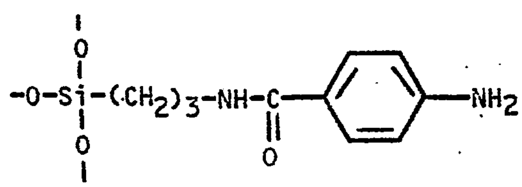

est décrit dans "Affinity Chromatography" pages 180–181, J. Turhova, Elsevier, 1978 pour une utilisation en chromatographie d'affinité mais pas pour une synthèse d'oligonucléotides.

L'invention a pour objet une application à la synthèse en phase solide d'oligonucléotides par la méthode aux phosphoramidites, aux phosphites, aux phosphodiesters et aux phosphotriesters, des supports de formule (I):

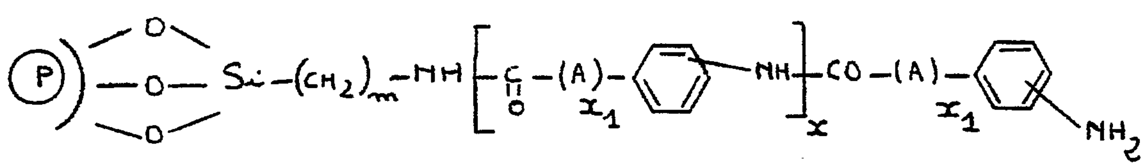

dans laquelle ® est un matériau constitué de microbilles de verre, de silice, de kieselguhr, de polytétrafluoroéthylène, d'oxydes métalliques ou de cellulose,

– m est un nombre entier pouvant varier de 1 à 20,

– A représente soit une chaîne alkyle linéaire ou ramifiée renfermant de 1 à 20 atomes de carbone, soit un radical cyclique saturé renfermant de 3 à 12 atomes de carbone, soit un radical phényle, soit un radical hétérocyclique renfermant 5 ou 6 chaînons,

– x est un nombre entier pouvant varier de 0 à 20,

– $x_1$ est un nombre entier pouvant varier de 0 à 10, les fonctions amines sur les noyaux phényles pouvant être en para, méta ou en ortho.

Dans la formule (I), lorsque A est une chaîne alkyle linéaire, A est représenté par le groupement $-(CH_2)_n-$, n étant un nombre entier pouvant varier de 1 à 20.

Lorsque A est une chaîne alkyle ramifiée, il s'agit de préférence d'une chaîne substituée par un ou plusieurs radicaux méthyle ou éthyle. Il s'agit, par exemple, de la chaîne méthyl-1 méthane diyl; méthyl-1 éthane diyl-1,2; méthyl-1 ou -2 propane diyl-1,3; méthyl-1,2 propane diyl-1,3; éthyl-1 éthane diyl-1,2.

Lorsque A représente un radical cyclique saturé, il s'agit de préférence du radical cyclopropane, cyclobutane, cyclopentane, cyclohexane, cycloheptane, cyclooctane, cyclononane, cyclodécane, cycloundécane, cyclododécane.

Lorsque A représente un radical cyclique saturé, il s'agit de préférence du radical cyclopropane, cyclobutane, cyclopentane, cyclohexane, cyclopheptane, cyclooctane, cyclononane, cyclodécane, cyclocindécane, cyclododécane.

Lorsque A représente un radical hétérocyclique renfermant 5 ou 6 chaînons, il s'agit de préférence du radical thiazolyle, pyridinyle, 4,5-dihydrothiazolyle, oxazolyle, isoxazolyle, imidazolyle, pyrimidyle, thiényle.

L'invention concerne notamment l'application à la synthèse d'oligonucléotides des supports de formule (I) pour lesquels ® est un matériau constitué de silice ayant une granulométrie homogène et pour lesquels les fonctions aminées sur les noyaux phényl sont soit en méta, soit en para.

On peut utiliser une silice commerciale, par exemple la silice VYDAC A® ayant des grains dont le diamètre est de 20 µm et des pores 300 Å.

Toute autre silice comparable à la silice VYDAC A® peut être utilisée.

On peut employer également une silice pour chromatographie, une silice HPLC, par exemple la silice commercialisée sous le nom de porosil B®, dont le diamètre des grains est compris entre 37 et 75 µm.

L'invention concerne notamment l'application des supports de formule (I), pour lesquels m est un nombre entier pouvant varier de 1 à 5 et pour lesquels A est un radical $-CH_2-$ et plus particulièrement de nouveaux supports de formule (I) pour lesquels x est un nombre entier pouvant varier 0 à 10 et $x_1$ un nombre entier pouvant varier de 0 à 5 et pour lesquels m = 3.

L'invention a tout particulièrement pour objet l'application des supports dont les formules suivent:

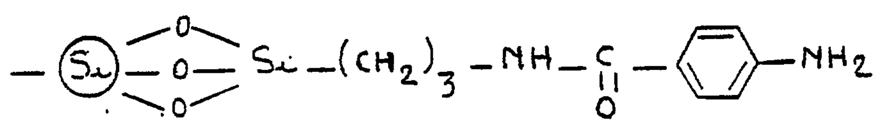

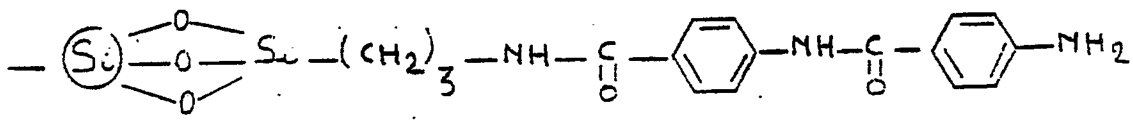

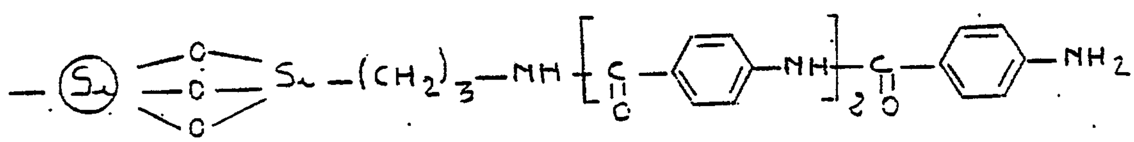

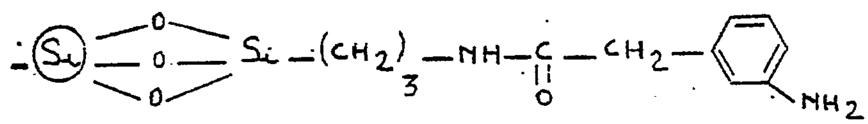

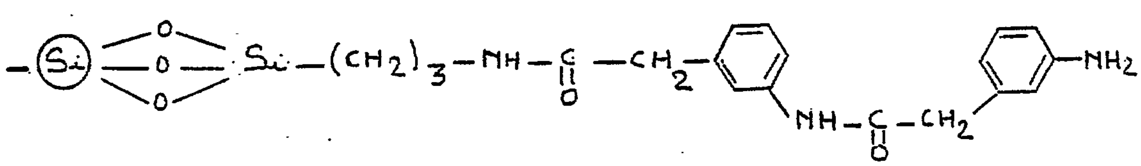

Le matériau 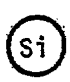 étant une silice VYDAC A® ou une silice équivalente.

Les supports de formule (I) sont préparés selon le procédé suivant:

On fait réagir un support de formule II:

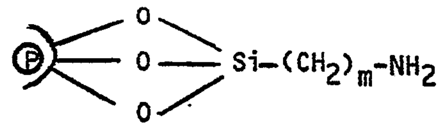 (II)

® et m ayant les significations déjà indiquées avec un composé de formule III:

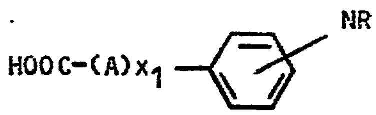 (III)

R étant un groupement protecteur de la fonction amine mono ou divalent et A et $x_1$ ayant les significations précédentes,
en présence d'un agent d'activation et
d'une base tertiaire, pour obtenir un support intermédiaire de formule:

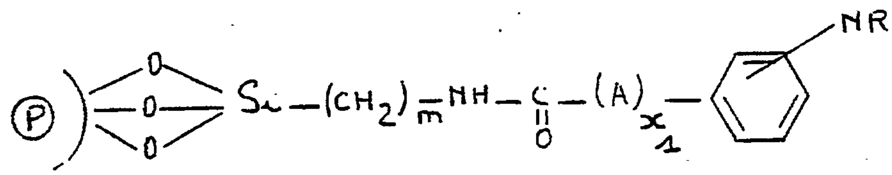

que l'on traite, selon la nature de R, par un acide ou une base pour libérer son groupement $-NH_9$ terminal et ainsi obtenir un support de formule I dans laquelle ®, m, A et $x_1$ ont les significations données précédemment et dans laquelle x = o, support de formule (I), que l'on traite de nouveau, le cas échant, par le composé de formule III dans les mêmes conditions que précédemment, pour obtenir un support intermédiaire de formule:

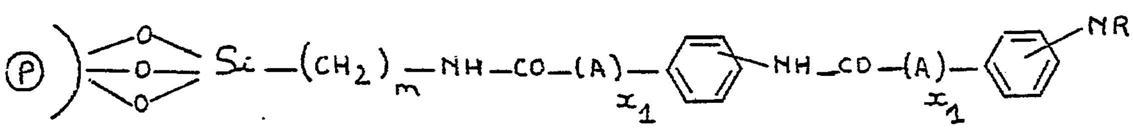

intermédiaire que l'on traite de nouveau par un acide ou une base pour obtenir un support de formule I dans laquelle x = 1, procédé que le cas échéant, l'on continue ainsi de suite en passant par des supports intermédiaires de formule :

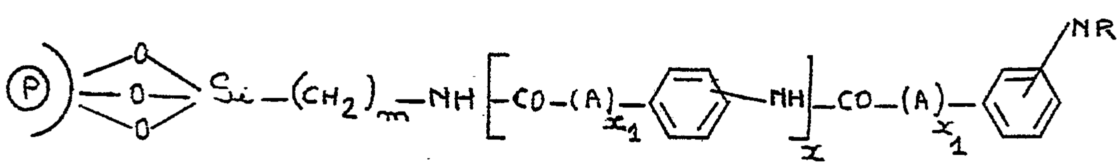

dans laquelle R, ®, m et $x_1$ ont les significations précédentes et dans laquelle x est un nombre entier pouvant varier de 0 à 20, jusqu'à obtention, le cas échant, d'un support de formule (I), dans laquelle x = 20.

Le groupement protecteur de la fonction amino R est par exemple un radical acyle dérivant d'un acide carbonique, tel que le radical éthoxy-carbonyle, benzyloxy-carbonyle, ter-butyloxy-carbonyle (= Boc), paraméthyloxy-benzyl oxy-carbonyle, fluorenyl méthoxy carbonyle (= FMOC). On peut aussi utiliser d'autres radicaux tels que des radicaux aryle ou aralkyle substitués ou non, par exemple les radicaux benzyle ou triphénylméthyle ou o-nitrophényl sulfényle.

Lorsque R a toutes les valeurs citées ci-dessus, la fonction amine est une amine secondaire, R est alors avantageusement une amine stable et notamment le groupement:

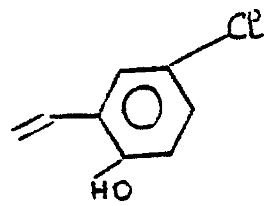

Il est évident que dans le cas où R est un radical monovalent, un atome d'hydrogène est lié à l'azote.

Dans le procédé donné ci-dessus R est de préférence le groupement:

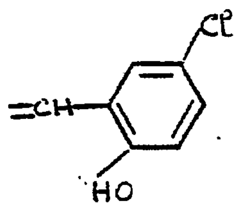

dans le composé de formule III.

Dans les conditions préférentielles de mise en œuvre du procédé:

– l'agent d'activation utilisé pour obtenir les supports intermédiaires est soit le phényl phosphoramido chloridate de phényle de formule:

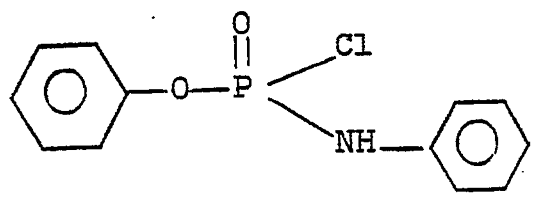

soit le dicyclohexylcarbodiimide.

– la base tertiaire est de préférence la triéthylamine ou la pyridine;

– la réaction s'effectue éventuellement au sein d'un solvant tel que le chlorure de méthylène, le diméthyl formamide, le tétrahydrofuranne, la pyridine.

Les conditions de déblocage de la fonction amine terminale des supports intermédiaires sont variables suivant les groupes protecteurs utilisés:

Lorsque R est un groupement:

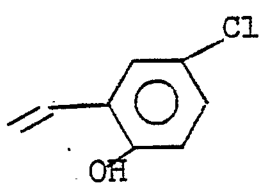

on utilise avantageusement l'acide chlorhydrique. D'autres acides tels que l'acide dichloroacétique ou trichloroacétique peuvent aussi être utilisés.

Les supports de formule II utilisés comme produits de départ dans le procédé de l'invention sont préparés comme indiqué dans le brevet européen n° 0 035 719.

Lorsque R représente le groupement:

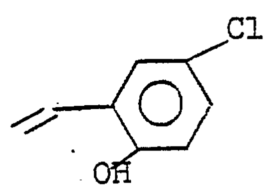

les produits de formule III sont préparés comme indiqué dans J.C. Sheemar et U.S. Grenida Am. Soc. 84 2457 1982 selon le schéma suivant:

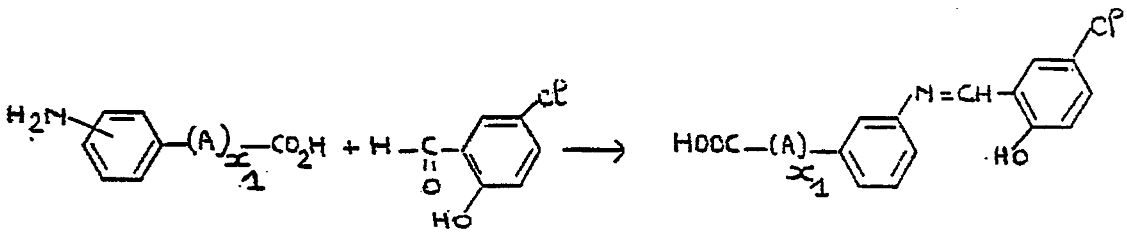

Les supports de formule (I) de l'invention permettent de réaliser, sans inconvénient, la synthèse d'oligonucléotides.

Les supports de formule (I) permettent notamment d'obtenir un titre élevé en premiers nucléosides et des intermédiaires stables. Ils peuvent être utilisés facilement dans les deux méthodes les plus classiques de synthèse solide d'oligonucléotides (méthode au phosphoramidite, au phosphite, au phosphodiester, au phosphotriester), aussi bien en 3'→5' qu'en 5'→3 et pour toutes les bases puriques ou pyrimidiques usuelles.

Par ailleurs, l'hydrolyse finale séparant le support du polynucléotide est réalisée facilement et en même temps que la déprotection des liaisons phosphates et des groupements protecteurs des bases puriques et pyrimidiques.

L'invention concerne tout particulièrement l'appliction des supports de formule (I) dans la synthèse solide par la méthode au phosphotriester.

L'invention a aussi pour objet les désoxyribonucléosides et ribonucléosides obtenus lors de la synthèse d'oligonucléotides mettant en jeu les supports de formule (I).

L'invention a ainsi pour objet de nouveaux désoxyribonucléosides et ribonucléosides sur supports de formule:

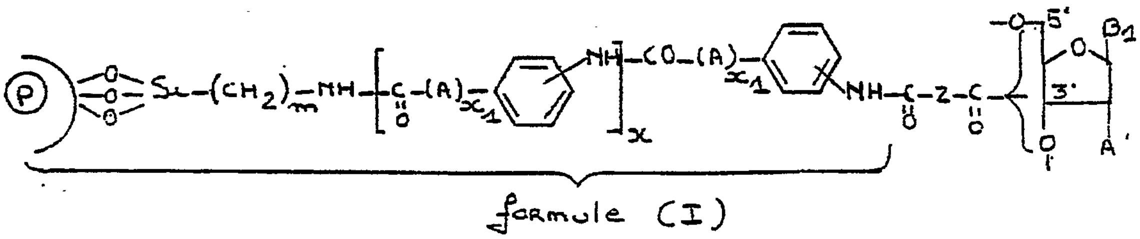

formule (I)

dans laquelle le support de formule (I) est relié à un ribonucléoside ou à un désoxyribonucléoside, soit en 3', soit en 5', par l'intermédiaire d'un groupement

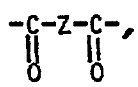

Z

étant un groupement hydrocarboné renfermant de 2 à 20 atomes de carbone, ou un groupement phényle, la fonction hydroxyle étant éventuellement protégée soit en 3', soit en 5' et dans laquelle A' est soit un atome d'hydrogène si le support de formule (I) est relié à un désoxyribonucléoside, soit $OR_1$ si le support de formule (I) est relié à un ribonucléoside, $R_1$ étant soit un atome d'hydrogène, soit un groupement protecteur usuel de la fonction hydroxyle, $B_1$ est une base purique ou pyrimidique dont la fonction amine est éventuellement protégée. L'invention a plus particulièrement pour objet de nouveaux désoxyribonucléosides sur supports de formule :

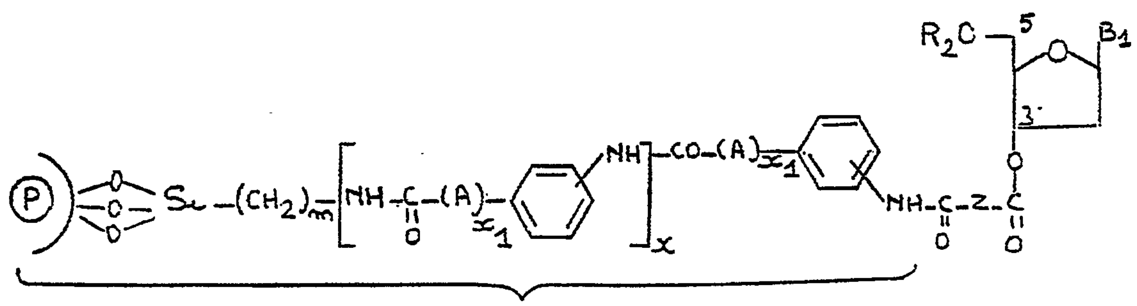

Formule (I)

dans laquelle le support de formule (I) est relié à un désoxyribonucléoside en 3' par l'intermédiaire d'un groupement

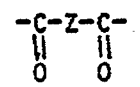

dans lequel Z est un groupement hydrocarboné renfermant de 2 à 20 atomes de carbone ou un groupement phényle et la fonction hydroxyle en 5' est éventuellement protégée par un groupement protecteur $R_2$ usuel, $B_1$ est une base purique ou pyrimidique, dont la fonction amine est éventuellement protégée.

L'invention concerne aussi les nucléotides obtenus par application des supports de formule (I) à leur synthèse.

L'invention a donc pour objet de nouveaux oligo désoxyribonucléotides ou oligoribonucléotides sur supports pour lesquels le nucléoside est attaché soit en 3', soit en 5', au support de formule (I), répond à la formule suivante :

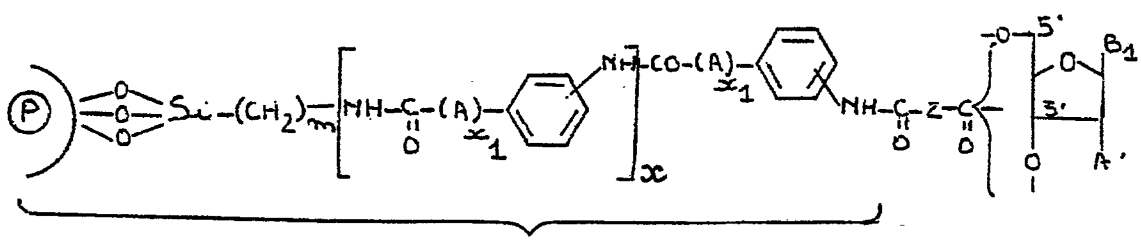

Formule (I)

et est lui-même relié par des liaisons phosphodiesters ou triesters du type :

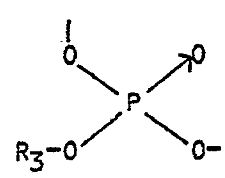

dans lesquelles $R_3$ est soit un atome hydrogène, soit un groupement protecteur, à d'autres nucléotides portant les bases $B_2$, .... B(y-1), jusqu'au dernier nucléoside de formule :

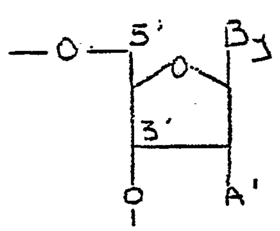

By étant la dernière base de l'oligodésoxy ou oligoribonucléotide, la fonction hydroxyle du dernier nucléoside en 5' ou en 3' étant éventuellement protégée et les différentes bases puriques ou pyrimidiques

ayant leurs fonctions amines éventuellement protégées. L'invention a plus particulièrement pour objet de nouveaux oligodésoxyribonucléotides sur supports de formule :

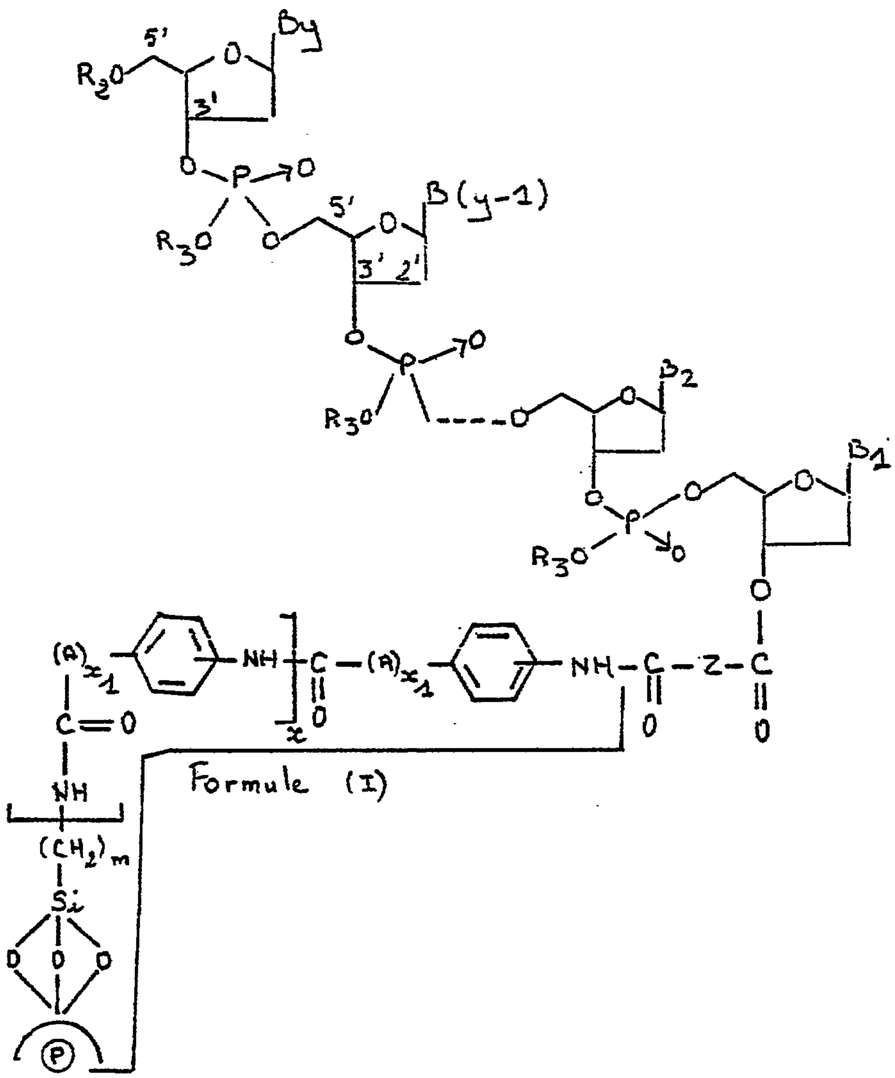


dans laquelle le support de formule (I) est relié en 3' à un oligodésoxyribonucléotide portant des bases $B_1$, $B_2$-----By et dans laquelle $R_2$ est soit un atome d'hydrogène, soit un groupement protecteur, $R_3$ est soit un atome d'hydrogène, soit un groupement protecteur, les bases puriques ou pyrimidiques ayant leurs fonctions amines éventuellement protégées.

Dans les désoxyribonucléosides, ribonucléosides, oligodésoxyribonucléotides ou oligoribonucléotides sur supports définis précédemment Z est de préférence un radical phényle ou un radical $-(CH_2)_n$, n étant un nombre entier pouvant varier entre 2 et 20.

L'invention concerne tout particulièrement ceux pour lesquels Z est un radical $-(CH_2)_2$.

Dans les ribonucléosides ou oligoribonucléosides, définis ci-dessus, $R_1$ est un groupement protecteur usuel de la fonction hydroxyle, tel qu'un groupement pyranyle, silyle ou benzyle.

Dans les nucléosides ou nucléotides définis précédemment, les bases $B_1$, $B_2$ --- By représentent l'adénine, la guanine (bases puriques), la cytosine, l'uracile ou la thymine (bases pyrimidiques).

Ces bases peuvent aussi être des bases puriques ou pyrimidiques substituées telles que par exemple la 6-méthylaminopurine ou la 6-diméthyl-aminopurine, la 1-méthyl guanine, la 5-méthyl-cytosine, la 5-hydroxyméthyl-cytosine, le dihydro-uracile.

Toutes les bases dites rares ou mineures que l'on trouve dans certains acides nucléiques peuvent être utilisées.

Les groupements protecteurs des fonctions amines de ces bases sont par exemple des groupements benzoyle ou isobutyryle.

Le groupement protecteur $R_2$ de la fonction hydroxyle en 5' est par exemple un radical trityle, monométhoxytrityle diméthoxytrityle, pixyle.

Le groupement protecteur $R_3$ des fonctions hydroxyle des groupements phosphate est par exemple le radical ortho ou para chlorophényle.

Les méthodes de synthèse d'oligonucléotides mentionnées précédemment sont très usuelles et parfaitement connues de l'homme de l'art. On peut les trouver résumées par exemple dans l'article The Chemical Synthesis of DNA, Aldrichimica Acta, vol. 16, N° 3-1983.

Il est rappellé brièvement ci-après les différentes étapes de la synthèse 3'→5' d'oligo désoxyribonucléotides par la méthode aux phosphotriesters. Il va de soi que les étapes sont strictement les mêmes pour la synthèse d'oligo ribonucléotides et/ou si la synthèse se fait en 5'→3', le groupement protecteur de l'hydroxyle en 3' devant être dans ce cas convenablement choisi.

1. <u>Préparation d'un désoxynucléoside activé:</u>

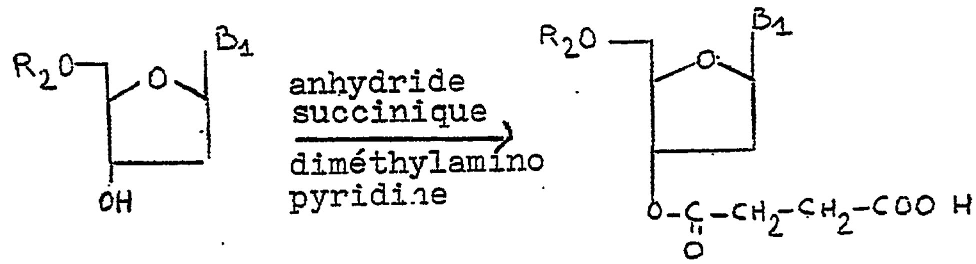


On peut utiliser l'acide libre ou activé par un groupement pentachloro phényle (Itakura et al. Nucl. Ac. Res. 8, 22, 5473, 1980) ou paranitro phényle : (MH Caruthers, Chemical and Enzymatic Synthesis of gene fragments H.Cr Gassen et A. Lang, Verlag Chemie (1982) p. 71).

2. <u>Condensation du désoxynucléoside préparé précédemment sur un support de formule (I) dénommé schématiquement ci-après:</u>

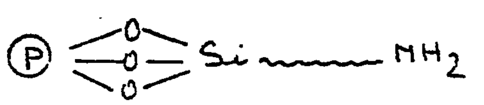

(I)

Cette condensation s'effectue en solution dans le diméthylformamide en utilisant comme catalyseur soit la triéthylamine pendant 20 à 24 heures, soit le dicyclohexylcarbodiimide dans la pyridine (1 nuit à 3 jours), soit la diméthylamino pyridine dans la pyridine.

On obtient :

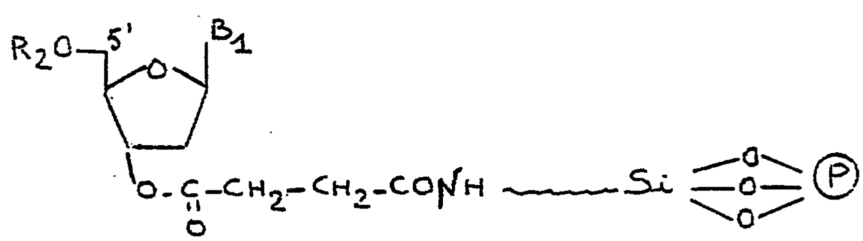

On déprotège la fonction hydroxyle en 5' par traitement avec un acide de Lewis, par exemple avec le bromure de zinc ou avec un acide di ou trichloroacétique.

3. <u>Allongement de la chaîne désoxyoligonucléotidique:</u>

On utilise soit des nucléotides monomères, soit des nucléotides dimères sous forme de leurs sels de triéthylammonium.

Les dimères sont stockés sous forme de dérivés cyanoéthylés. On prépare le sel de triéthylammonium juste avant la synthèse.

Après avoir éliminé le groupement protecteur de l'hydroxyle 5' du premier nucléoside fixé sur le support, on condense ce dimère en présence de mésitylsulfonyl 3-nitro 1, 2, 4-triazole ou MSNT ou du mélange chlorure de mésitylsulfonyl-méthyl imidazole dans la pyridine.

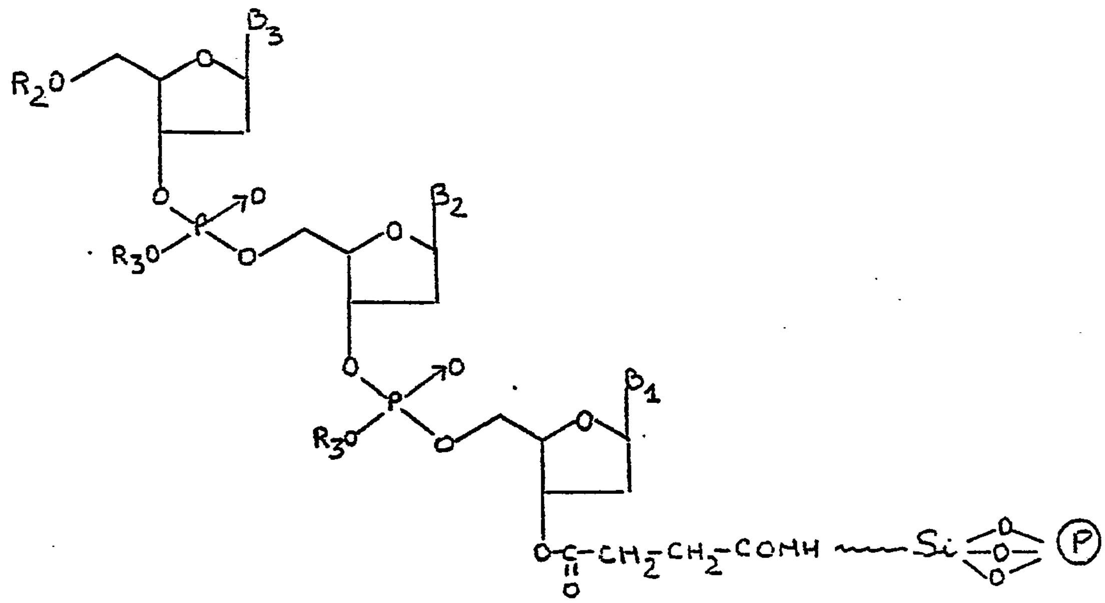

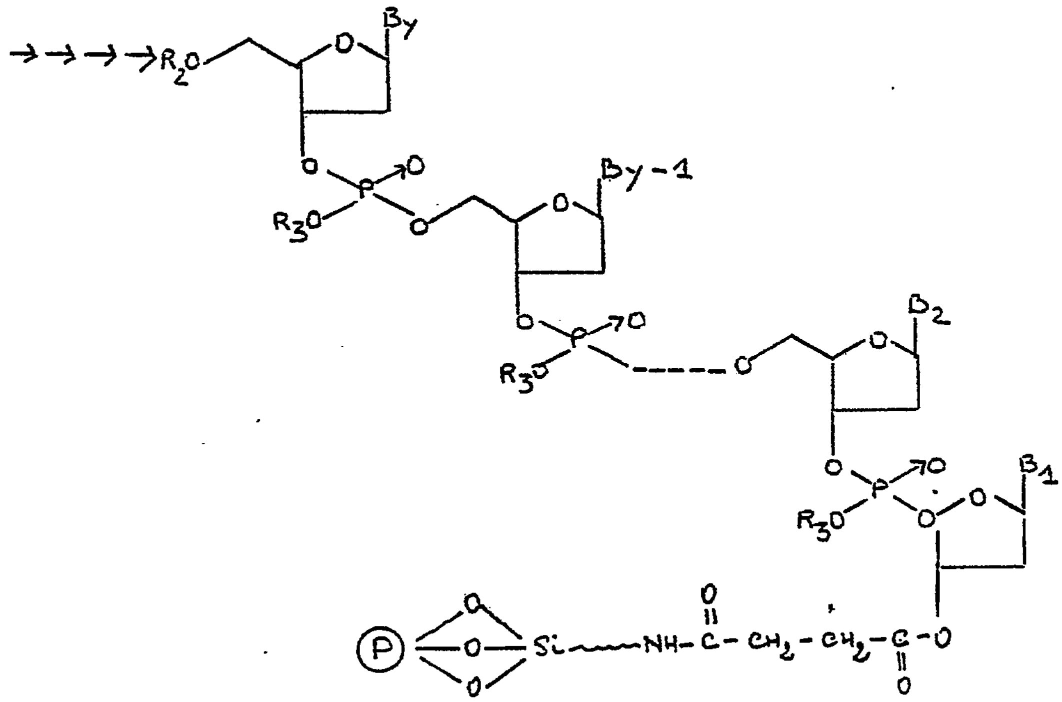

Avant chaque couplage, on élimine le groupement protecteur en 5' du dernier nucléotide accroché sur la chaîne.

4. Séparation du support et de la chaîne oligonucléotidique

On utilise, par exemple, le mélange para-nitrobenzaldoxime et N,N,N',N'-tétraméthylguanidine dans le mélange dioxane et eau (1/1) qui coupe l'oligonucléotide du support.

Ce rèactif très doux permet aussi de cliver sélectivement les liaisons phosphates aryliques par rapport aux phosphates aliphatiques réalisant ainsi le déblocage des phosphates sans casser la chaîne synthétisée.

Il faut ensuite traiter l'oligonucléotide ainsi obtenu, de façon à libérer toutes les fonctions protégées lors de la synthèse.

Plusieurs traitements de purification (électrophorèse HPLC) sont alors nécessaires pour obtenir le polynuclèotide désiré.

Seule, une ultime étape de séquençage permet de connaître de façon certaine, si cela s'avére nécessaire, la structure de l'oligonucléotide.

Les exemples donnés ci-dessous illustrent l'invention, sans toutefois la limiter.

Exemple 1: Préparation du support:

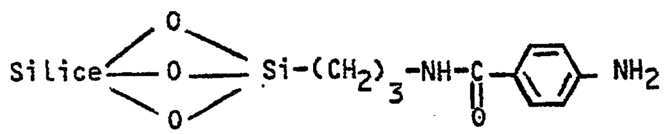

STADE A: Préparation du support de départ:

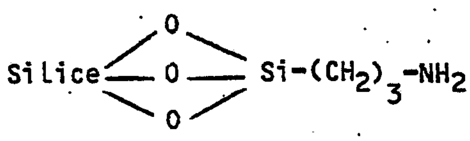

On opère comme indiqué au début de l'exemple 1 du brevet européen nº 0035719 à partir de 10 g de silice VYDAC A® (granulométrie 20 µ--Pores 300 Å) et de 11,5 g de 3-amino propyltriéthoxy silane.

On obtient le support attendu présentant un titre en $NH_2$ de $3.10^{-4}$ équivalent/gramme (dosage par la méthode à l'acide picrique).

STADE B: Préparation du support.

On agite pendant une nuit :
40 - 600 mg de support obtenu au stade A,
- 550 mg d'acide 4 /(5-chloro 2-hydroxy benzylidène)-amino/benzoïque (préparation donnée à la fin de l'exemple 1),
- 534 mg de phényl phosphoramido chloridate de phényle (préparation donnée à la fin de l'exemple 1),
- 10 cm3 de chlorure de méthylène,
- 0,54 cm3 de triéthylamine.

On essore le précipité obtenu, empâte avec du diméthylformamide chaud (100°C), jusqu'à absence de coloration du filtrat. On effectue sur la silice un test à la ninhydrine qui est négatif (absence de groupement $-NH_2$ libre).

On obtient un support pesant 583 mg que l'on met en suspension dans 5 cm3 de méthanol à 50% dans l'eau. On ajoute de l'acide chlorhydrique 0,1 N pour maintenir le pH à 1, agite 1 heure, essore, lave successivement avec :
- 20 cm3 d'un mélange méthanol-eau (1-1),
- 100 cm3 de méthanol anhydre,
- 100 cm3 de chlorure de méthylène.

On sèche sous pression réduite, à température ambiante pendant une nuit et obtient 563,5 mg de support attendu.

1/. Préparation de l'acide 4 -/(5-chloro 2-hydroxy benzylidène) amino/benzoïque.

On opère comme indiqué dans J.C. Sheenam et U.S. Grenda Am. Soc. 84 2457 (1982) à partir de 1,37 g d'acide para-amino benzoïque, de 2,35 g de 5 chloro-salicylaldéhyde, de 240 cm3 d'éthanol et de 17 cm3 de méthanol anhydre.

On obtient 2,59 g de produit attendu.

2/. Préparation du phényl phosphoramido chloridate de phényle.

On opère comme indiqué dans Synthésis 288 (1982) à partir de 22,2 cm3 de phosphorodichloridate de phényle, de 175 cm3 de benzène anhydre, d'une solution renfermant 12,5 cm3 d'aniline dans 75 cm3 de benzène anhydre.

On obtient 24,8 g de produit attendu.

Exemple 2: Préparation du support :

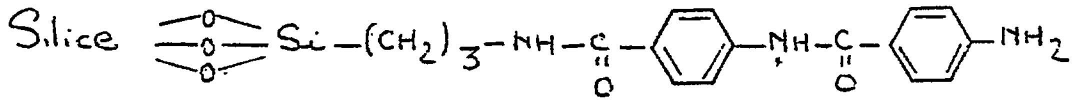

On opére comme au stade B de l'exemple 1, à partir du support obtenu à l'exemple 1 et obtient 490 mg de support attendu ayant un titre en $NH^2$ de $2.10^{-4}$ équivalent/gramme (dosage à l'acide picrique).

Exemple 3: Préparation de support.

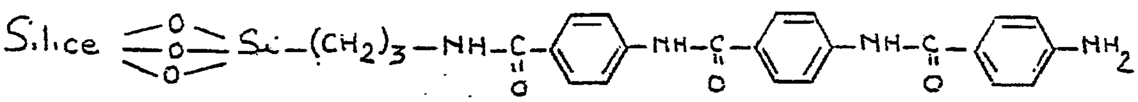

On opère comme au stade B de l'exemple 1, à partir du support obtenu à l'exemple 2.

On obtient 462 mg de support attendu ayant un titre en $NH_2$ de $1,5.10^{-4}$ équivalent/gramme (dosage à l'acide picrique).

Exemple 4 : Préparation du support.

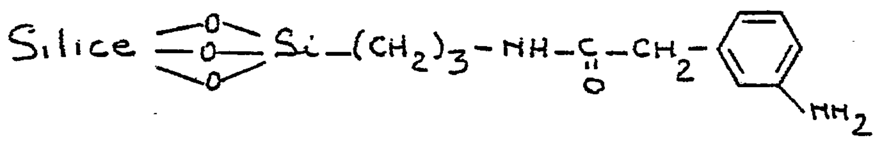

On agite pendant 48 heures à température ambiante :

- 200 mg de support obtenu au stade A de l'exemple 1,
- 5 cm3 de pyridine anhydre,
- 275 mg d'acide 3 [(5-chloro 2-hydroxy benzylidène) amino] phényl acétique, (préparation donnée à la fin de l'exemple 4),
- 200 mg de dicyclohexyl carbodiimide.

On essore, puis lave successivement avec du dioxane, du méthanol, du diméthylformamide chaud, du méthanol.

On sèche sous pression réduite à température ambiante et obtient 195 mg de support attendu.

Le test à la ninhydrine est négatif.

La silice précédemment obtenue est mise en suspension dans 2 cm3 d'acide chlorhydrique 0,1 N, 5 cm3 de méthanol, 3 cm3 d'eau. On agite 1 heure à température ambiante, essore, lave avec le mélange méthanol-eau (1-1).

On obtient 190 mg de support attendu ayant un titre en $-NH_2$ de $1,95.10^{-4}$ équivalent/gramme (titre obtenu par dosage photométrique en UV du 2-hydroxy 5-chloro benzaldéhyde libéré contenu dans le filtrat).

Préparation de l'acide 3-[(5-chloro 2-hydroxybenzylidène) amino] phényl acétique.

On opère comme indiqué dans J.C. Sheemann Am. Soc. 84 2457 (1982) à partir de 1,5 g d'acide 3-aminophényl acétique, de 1,72 g de 5-chloro 2-hydroxy benzaldéhyde, de 480 cm3 d'éthanol 100° et de 35 cm3 de méthanol.

On obtient 2 g de produit attendu.

Exemple 5: Préparation du support

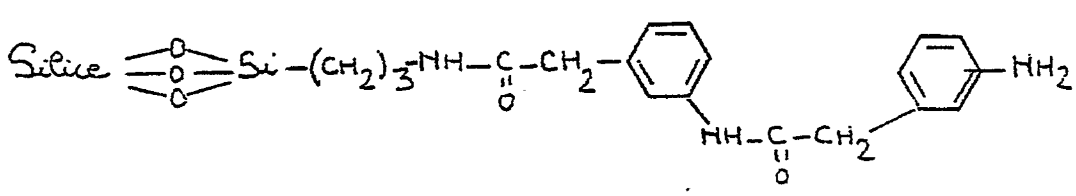

On agite pendant 72 heures le support obtenu à l'exemple 4 avec 5 cm3 de pyridine anhydre, 200 mg

d'acide 3-[(5-chloro 2-hydroxy benzylidène) amino] phényl acétique et 200 mg de dicyclohexyl carbodiimide.

On essore et lave successivement avec du diméthylformamide, du méthanol, du chlorure de méthylène, de l'oxyde d'éthyle. On sèche sous pression réduite. On reprend le produit obtenu avec 2 cm3 d'acide chlorhydrique 0,1 N, 5 cm3 de méthanol, 3 cm3 d'eau, agite 1 heure, essore, lave avec un mélange méthanol-eau (1-1). On obtient 180 mg de support attendu et ayant un titre en $NH_2$ de $1,97.10^{-4}$ équivalent/gramme (titre obtenu par dosage photométrique en UV du 2-hydroxy 5-chloro benzaldéhyde libéré contenu dans le filtrat).

Synthèse d'oligonucléotides par la méthode au phosphotriester

Le support de formule (I) est disposé dans une minicolonne entre deux filtres en polyfluoroéthylène. Les deux filtres sont maintenus dans une position fixe par deux pistons creux. L'ensemble est fermé en haut par un bouchon à vis muni d'un septum par lequel on introduit à la seringue le mélange de couplage. L'appareil utilisé est semblable à celui décrit dans Chemical and Enzymatic Synthésis of fragments H.Cr Gassen A. Lang Verlag Chemie 82, p. 14.

Toutes les opérations répétitives de lavage et d'introduction de réactifs sont automatisées. Le nombre de nucléotides à introduire pour la totalité de la synthèse peut être programmé. L'introduction du nucléotide est la seule opération manuelle à effectuer à la seringue.

On place dans le réacteur la quantité de support de formule (I) condensé avec le premier nucléoside portant la première base $B_1$ nécessaire pour la synthèse envisagée (25 à 150 mg). On programme convenablement les paramètres souhaités, en particulier le nombre de nucléotides à accrocher, puis on commence le cycle automatique suivant:

Etape 1:

| Solvant | Type d'introduction | Temps |
|---|---|---|
| Pyridine | Flux continu 1 $cm^3$/mn | 5 mn |
| Isocyanate de phényle à 10% dans la pyridine | Fractions programmées | 10 mn |
| Pyridine | Flux continu 1 $cm^3$/mn | 5 mn |
| Chlorure de méthylène | Flux continu environ 2 $cm^3$/mn | 3 mn |
| acide dichloro acétique à 10% dans le chlorure de méthylène | Flux continu environ 2 $cm^3$/mn | 4,5 mn |
| DMF | Flux continu environ 1 $cm^3$/mn | 5 mn |
| Pyridine | Flux continu environ 1 $cm^3$/mn | 5 mn |
| Couplage | à la seringue | 15 mn à 1 h |

Etape 2:

On effectue autant de cycles identiques au premier, qu'il est nécessaire pour obtenir le nucléotide souhaité.

Les solvants utilisés dans ce cycle de synthèse doivent être très purs et anhydres.

Le dosage UV de la quantité des ions tritylium effectué après détritylation permet de connaître le rendement de chaque couplage.

Le mélange de couplage est préparé juste au moment de l'emploi, il comprend :

- 10 équivalents de sels de triéthylammonium du nucléotide monomère ou dimère (par rapport à la quantité du premier nucléoside présent sur le support solide).
- 30 équivalents de mésityl sulfonyl 3-nitro 1,2,4-triazole ou MSNT dans la pyridine anhydre (0,3 cm3 pour 50 mg de dimère environ).

Ce mélange est transféré dans une seringue sous atmosphère d'argon anhydre, on l'ajoute en trois fois à intervalles réguliers.

Pour obtenir l'oligonucléotide totalement déprotégé, il est ensuite nécessaire d'effectuer un certain nombre de traitements qui sont très usuels et décrits, par exemple, dans Chemical and Enzymatic Synthesis of gene fragments H. Cr. Gassen and A. Lang Verlag Chemie 82, pages 2 à 42.

Ces traitements sont les suivants :

1/. Cliver le nucléotide de son support solide.

On effectue un traitement par une solution 0,3 M d'o-nitrobenzaldoximate de 1, 1, 3, 3-tétraméthyl guanidinium dans un mélange dioxane-eau 1-1.

On procède de manière très usuelle, comme indiqué dans Nucleic Acids Research. Vol. 9 n° 18 1981, p. 4611.

Ce réactif très doux permet aussi de cliver sélectivement les liaisons phosphates aryliques par rapport aux phosphates aliphatiques réalisant ainsi le déblocage des phosphates sans casser la chaîne synthétisée.

2/. Débloquer les fonctions amines des bases azotées.

On effectue un traitement par $NH_4OH$ saturé ( 37%) et libère ainsi toutes les amines des bases azotées.

3/. Débloquer la fonction en 5' du dernier nucléotide.

On effectue un traitement par un mélange $CH_3COOH$-eau (4-1).

Après concentration à sec, sous pression réduite, on reprend avec de l'eau et extrait à l'éther pour éliminer tous les réactifs et les produits de clivage.

L'oligonucléotide à y maillons ainsi obtenu, contient de nombreuses impuretés (nucléotides à y-2, y-4--- maillons, produits de diverses dégradations).

L'obtention du produit recherché nécessite plusieurs étapes successives de purification :

- chromatographie sur gels,
- HPLC,
- Electrophorèse,
- Séquençage : Cette dernière méthode donne sans ambiguité, dans l'ordre, la suite des nucléotides monomères.

Ces méthodes sont très usuelles.

La demanderesse ne fait donc que les citer.

Exemple 6: Oligo-désoxyribonucléotides synthétisés en utilisant le support de l'exemple 2.

1/. Préparation du 5'-diméthoxytrityl 2'-déoxy thymidine 3'-paranitro phényl succinate

On utilise la méthode décrite par MH Caruthers, Chemical and Enzymatic Synthetis of gene fragments, H.Cr. Gassen et A Lang Verlag Chemie (1982) p. 71, à partir de 1,557 g d'acide 5'-diméthoxytrityle 2'-déoxy thymidine 3'-succinique, de 10 cm3 de dioxane anhydre, de 0,5 cm3 de pyridine anhydre, de 369 mg de para nitrophénol, de 585 mg de dicyclohexyl carbodiimide en solution dans 2,5 cm3 de dioxane anhydre.

On obtient 1,150 g de produit attendu.

2/. Condensation entre le support de l'exemple 2 et le succinate de thymidine activé préparé ci-dessus.

On agite à l'obscurité, pendant 72 heures, 99 mg de support de l'exemple 2 titrant en $NH_2$ $2.10^{-4}$ eq/g, 80 mg de succinate de thymidine activé préparé ci-dessus, soit environ 6,3 équivalents, 80 mg de dicyclohexyl carbodiimide, 2 cm3 de pyridine. On essore et lave successivement avec de la pyridine, un mélange chlorure de méthylèneméthanol,du chlorure de méthylène, puis sèche sous pression réduite.

On obtient 95 mg de support condensé attendu, ayant un titre en diméthoxytrityl de 8,8 $10^{-5}$ eq/g.

3/. On obtient, à partir du support condensé avec le succinate de thymidine, en utilisant trois monomères, l'oligo-désoxyribonucléotide 5'-d (T C T A) et en utilisant trois dimères, l'oligo-désoxyribonucléotide 5'-d (TCC TT AC).

Exemple 7: oligo-désoxyribonucléotide synthétisé en utilisant le support de l'exemple 3.

1/. Préparation du 5'-diméthoxytrityl 2'-N benzoyl désoxycytidine 3'-para nitro phényl succinate.

On utilise la méthode décrite par MH Caruthers, Chemical and Enzymatic Synthesis of gene fragments, H-Cr Gassen et A. Lang, Verag Chemie (1982) p. 71, à partir de 4,16 g d'acide 5'-diméthoxytrityl 2'-déoxy N-benzoyl cytidine 3'-succinique, 25 cm3 de dioxane anhydre, 0,8 cm3 de pyridine, 1,16 g de paranitrophénol, 1,83 g de dicyclohexylcarbodiimide dans 8,33 cm3 de pyridine.

On obtient 3,6 g de produit attendu.

2/. Codensation entre le support de l'exemple 3 et le succinate de cytosine activé préparé ci-dessus.

On mélange pendant deux jours à l'obscurité 100 mg de support de l'exemple 3 titrant en $NH_2$ 1,5 $10^{-4}$ eq/g, 110 mg de succinate de cytosine activé préparé ci-dessus, soit environ 10 équivalents et 87 mg de dicyclohexylcarbodiimide. On lave et sèche comme à l'exemple précédent et obtient 95 mg de support condensé attendu ayant un titre en diméthoxytrityle de $7\text{-}10^{5}$ eq/g.

3/. On obtient, à partir du support condensé avec le succinate de cytosine en utilisant 3 dimères, l'oligodésoxyribonucléotide 5'-d(CCC TTAC).

Exemple 8: oligo désoxyribonucléotides synthétisés en utilisant le support de l'exemple 5.

On condense 145 mg de support de l'exemple 5 avec 220 mg de succinate de cytosine activé en présence de 150 mg de dicyclohexyl carbodiimide et de 2 cm3 de pyridine de la même façon qu'à l'exemple 7. On obtient 140 mg de support condensé attendu titrant 5,55 $10^5$ eq/g en diméthoxytrityle.

On obtient à partir de ce support condensé en utilisant 3 dimères, l'oligodésoxyribonucléotide 5'-d (CCC TTAC) et en utilisant deux dimères, l'oligodésoxyribonucléotide 5'-d (CCCTT).

**Revendications**

1. Application à la synthèse en phase solide d'oligonucléotides par la méthode aux phosphoramidites, aux phosphites, aux phosphodiesters et aux phosphotriesters, des supports de formule (I):

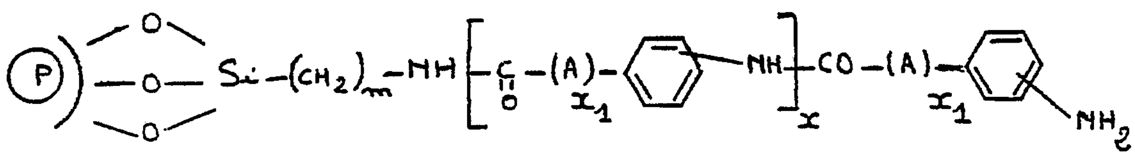

(I)

dans laquelle ® est un matériau constitué de microbilles de verre, de silice, de kieselguhr, de polytétrafluoroéthylène, d'oxydes métalliques ou de cellulose,

– m est un nombre entier pouvant varier de 1 à 20,

– A représente soit une chaîne alkyle linéaire ou ramifiée renfermant de 1 à 20 atomes de carbone, soit un radical cyclique saturé refermant de 3 à 12 atomes de carbone, soit un radical phényle, soit un radical hétérocyclique renfermant 5 ou 6 chaînons,

– x est un nombre entier pouvant varier de 0 à 20,

– $x_1$ est un nombre entier pouvant varier de 0 à 10, les fonctions amines sur les noyaux phényl pouvant être en para, méta ou en ortho.

2. Application selon la revendication 1, des supports de formule (I) pour lesquels ® est un matériau constitué de silice ayant une granulométrie homogène et pour lesquels les fonctions aminées sur les noyaux phényl sont soit en méta, soit en para.

3. Application selon les revendications 1 et 2, des supports de formule (I) pour lesquels m est un nombre entier pouvant varier de 1 à 5.

4. Application selon les revendications 1 à 3, des supports de formule (I), pour lesquels A est un radical $-CH_2-$.

5. Application selon les revendications 1 à 4 des supports de formule (I) pour lesquels x est un nombre entier pouvant varier de 0 à 10 et $x_1$ un nombre entier pouvant varier de 0 à 5.

6. Application selon les revendications 1 à 5 des supports de formule (I) pour lesquels m = 3.

7. Application selon les revendications 1 à 6 des supports dont les formules suivent:

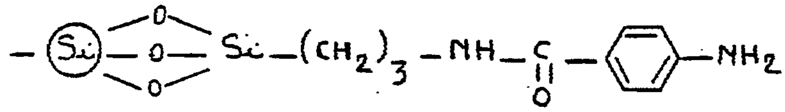

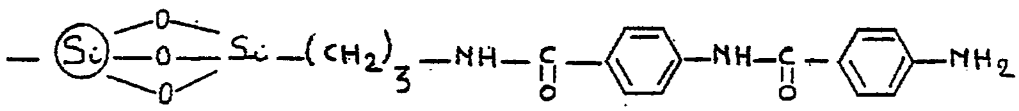

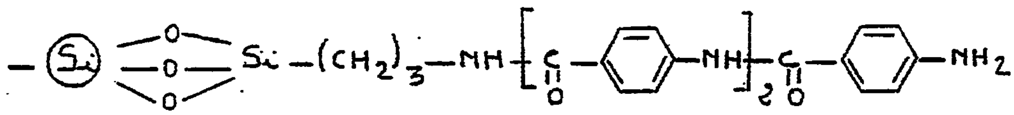

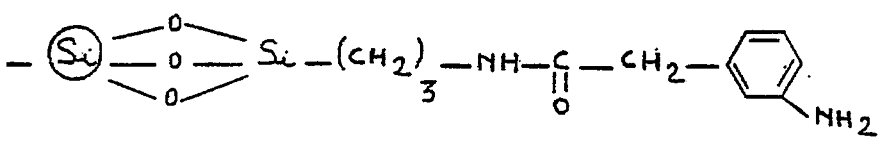

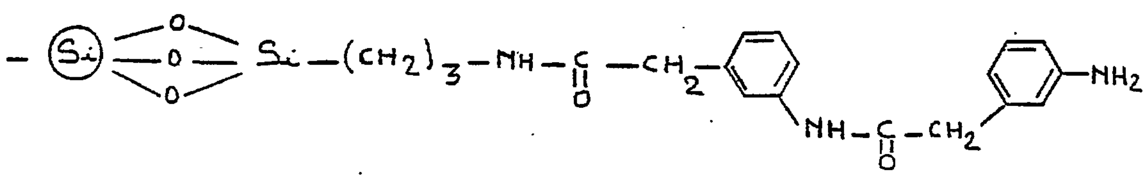

le matériau Si étant une silice VYDAC A® ou une silice équivalente.

8. Application des supports de formule (I) à la synthèse en phase solide d'oligonculéotides, par la méthode aux phosphotriesters.

9. Nouveaux désoxyribonucléosides ou ribonucléosides sur supports de formule:

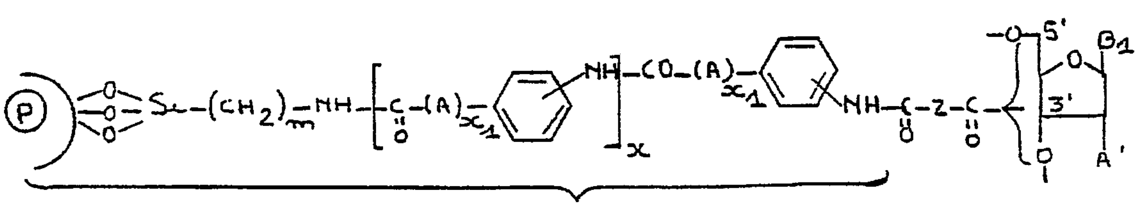

formule (I)

dans laquelle le support de formule (I) est relié à un ribonucléoside ou à un désoxyribonucléoside, soit en 3', soit en 5', par l'intermédiaire d'un groupement

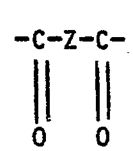

Z étant un groupement hydrocarboné renfermant de 2 à 20 atomes de carbone, ou un groupement phényl, la fonction hydroxyle étant éventuellement protégée, soit en 3′, soit en 5′ et dans laquelle A′ est soit un atome d'hydrogène si le support de formule (I) est relié à un désoxyribonucléoside, soit $OR_1$ si le support de formule (I) est relié à un ribonucléoside, $R_1$ étant soit un atome d'hydrogène, soit un groupement protecteur usuel de la fonction hydroxyle, $B_1$ est une base purique ou pyrimidique dont la fonction amine est éventuellement protégée.

10. Nouveaux désoxyribonucléosides sur supports, tels que définis à la revendication 9, de formule:

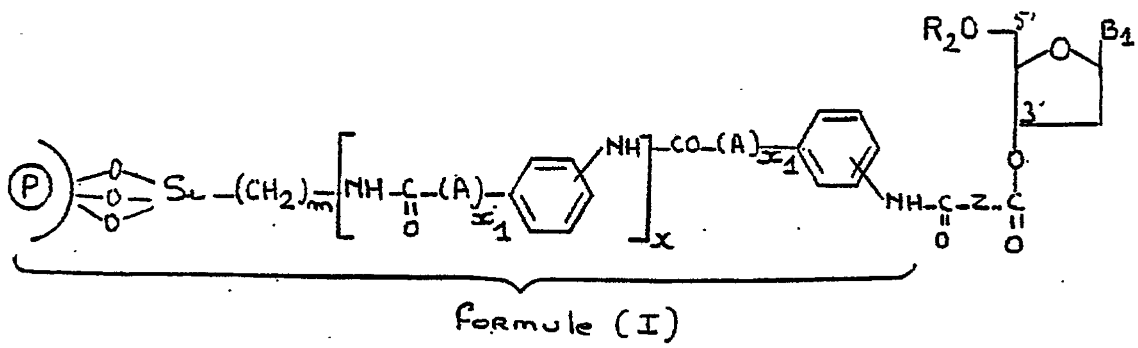

dans laquelle le support de formule (I) est relié à un désoxyribonucléoside en 3′ par l'intermédiaire d'un groupement

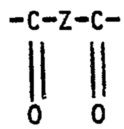

dans lequel Z est un groupement hydrocarboné renfermant de 2 à 20 atomes de carbone ou un groupement phényle et la fonction hydroxyle en 5′ est éventuellement protégée par un groupement protecteur $R_2$ usuel, $B_1$ est une base purique ou pyrimidique, dont la fonction amine est éventuellement protégée.

11. Nouveaux oligo désoxyribonucléotides ou oligoribonucléotides sur supports pour lesquels le nucléoside est attaché soit en 3′, soit en 5′, au support de formule (I), répond à la formule suivante:

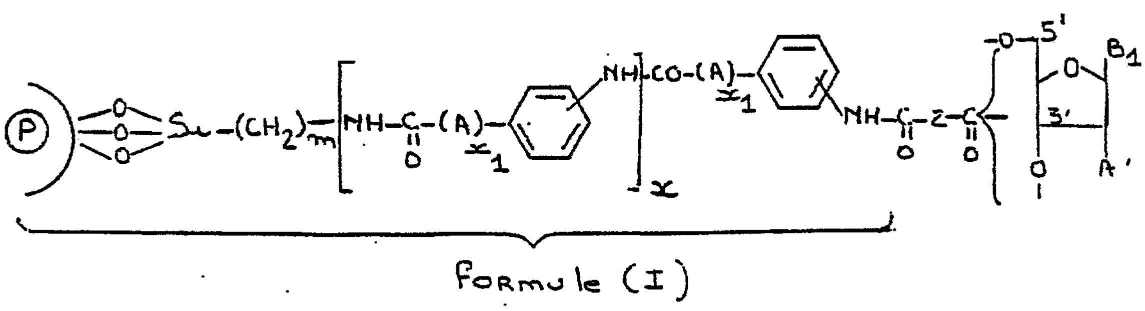

et est lui-même relié par des liaisons phosphodiesters ou triesters du type:

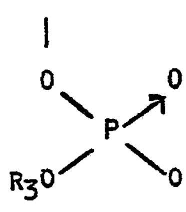

dans lesquelles $R_3$ est soit un atome d'hydrogène, soit un groupement protecteur, à d'autres nucléotides portant les bases $B_2$, ... B(y−1) jusqu'au dernier nucléoside de formule:

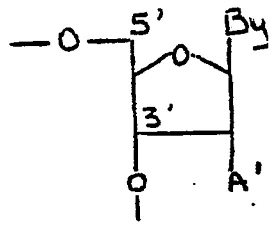

By étant la dernière base de l'oligodésoxy ou oligoribonucléotide, la fonction hydroxyle du dernier nucléoside en 5′ ou en 3′ étant éventuellement protégée et les différentes bases puriques ou pyrimidiques ayant leurs fonctions amines éventuellement protégées.

12. Nouveaux oligodésoxyribonucléotides sur supports, tels que définis à la revendication 11, de formule:

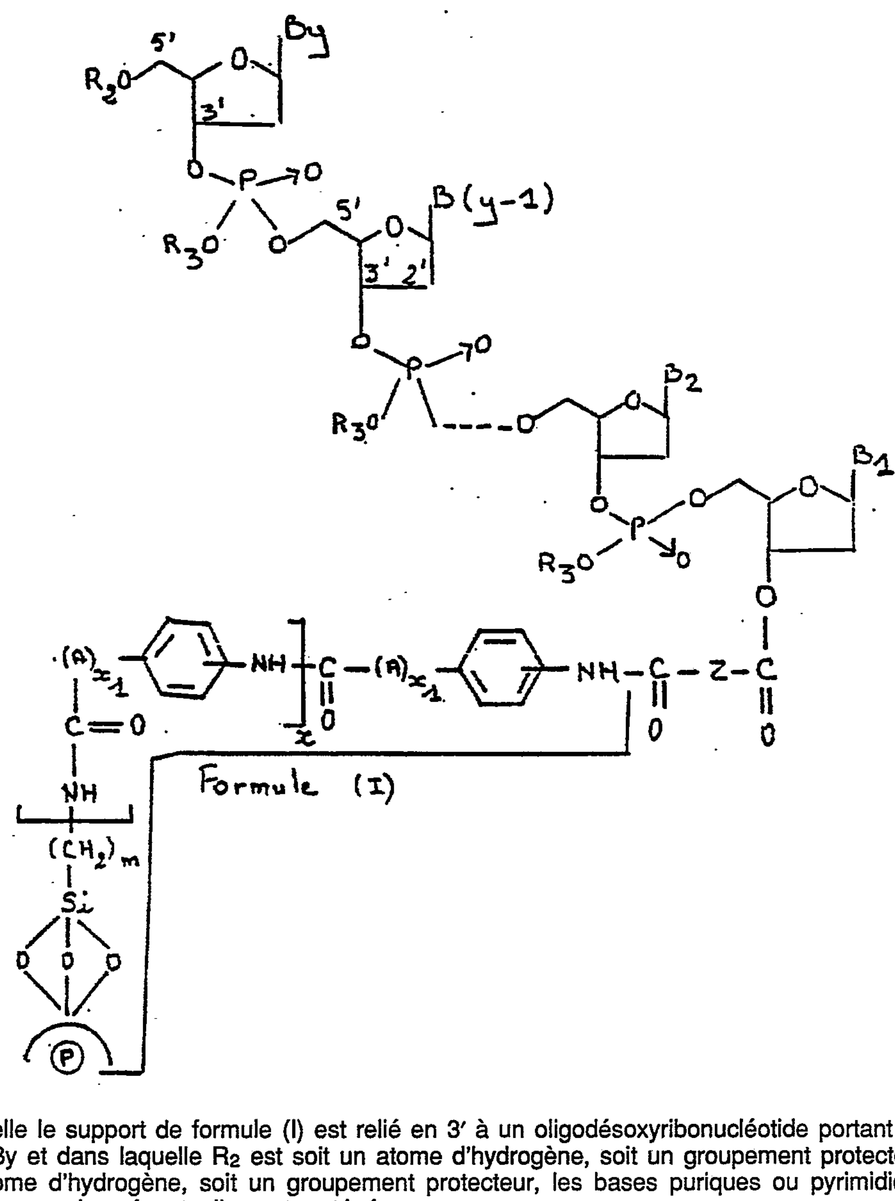

dans laquelle le support de formule (I) est relié en 3′ à un oligodésoxyribonucléotide portant des bases $B_1$, $B_2$-----By et dans laquelle $R_2$ est soit un atome d'hydrogène, soit un groupement protecteur, $R_3$ est soit un atome d'hydrogène, soit un groupement protecteur, les bases puriques ou pyrimidiques ayant leurs fonctions amines éventuellement protégées.

13. Nouveaux désoxyribonucléosides, ribonucléosides, oligodésoxyribonucléotides ou oligoribonucléotides sur supports tels que définis aux revendictions 9 à 12 pour lesquels Z est le radical $-(CH_2)_2-$.

## Patentansprüche

1. Verwendung bei der Synthese von Oligonucleotiden durch die Methode mit Phosphoramiditen, Phosphiten, Phosphodiestern und Phosphotriestern in fester Phase der Träger der Formel (I)

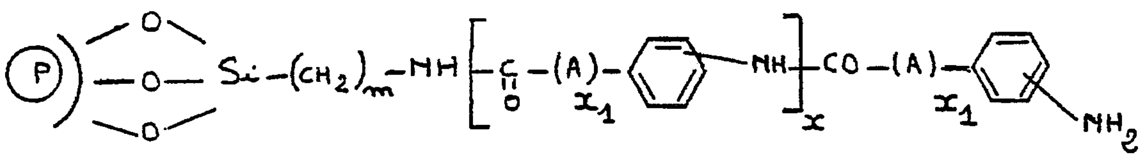

(I)

worin ® ein Material ist, bestehend aus Mikrokügelchen aus Glas, Siliciumdioxid, Kieselgur, Polytetrafluorethylen, Metalloxiden oder Cellulose,
m für eine Zahl von 1 bis 20 steht,
A entweder eine lineare oder verzweigte Alkylkette mit 1 bis 20 Kohlenstoffatomen oder einen gesättigten, cyclischen Rest mit 3 bis 12 Kohlenstoffatomen oder einen Phenylrest oder einen heterocyclischen Rest mit 5 oder 6 Kettengliedern wiedergibt,
x für eine ganze Zahl von 0 bis 20 steht,
$x_1$ für eine ganze Zahl von 0 bis 10 steht,
wobei die Aminfunktionen an den Phenylkernen sich in para-, meta- oder ortho-Stellung befinden können.

2. Verwendung gemäß Anspruch 1 der Träger der Formel (I), worin ® für ein Material steht, bestehend aus Siliciumdioxid mit einer homogenen Granulometrie, und deren Aminfunktionen an den Phenylkernen sich entweder in meta- oder in para-Stellung befinden.

3. Verwendung gemäß den Ansprüchen 1 und 2 der Träger der Formel (I), worin m für eine ganze Zahl von 1 bis 5 steht.

4. Verwendung gemäß den Ansprüchen 1 bis 3 der Träger der Formel (I), worin A einen Rest $-CH_2-$ wiedergibt.

5. Verwendung gemäß den Ansprüchen 1 bis 4 der Träger der Formel (I), worin x für eine ganze Zahl von 0 bis 10 steht und $x_1$ für eine ganze Zahl von 0 bis 5 steht.

6. Verwendung gemäß den Ansprüchen 1 bis 5 der Träger der Formel (I), worin m = 3.

7. Verwendung gemäß den Ansprüchen 1 bis 6 der Träger der folgenden Formeln:

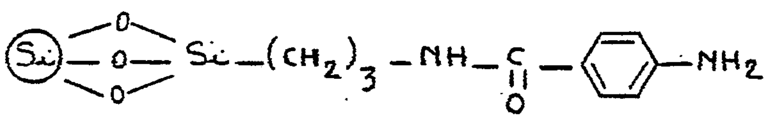

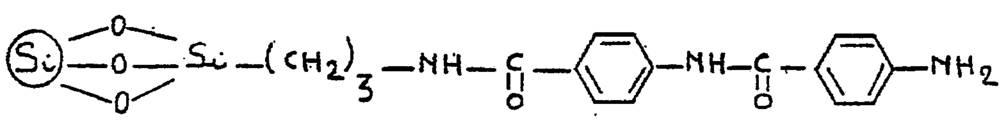

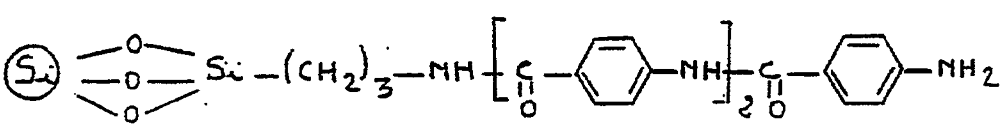

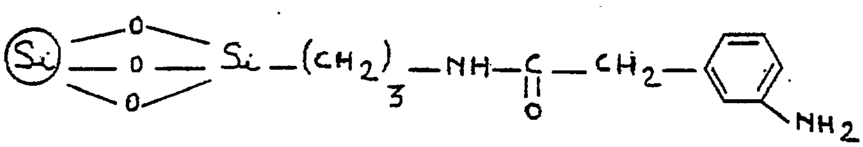

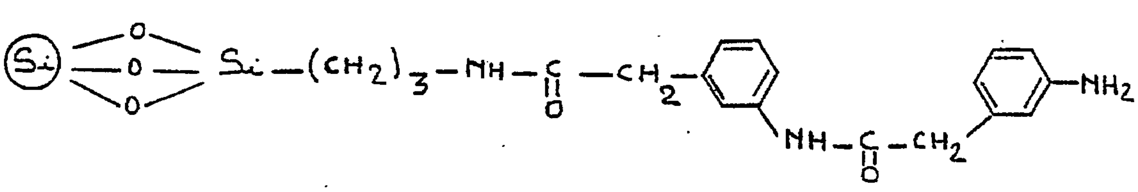

wobei das Material Si ein Siliciumdioxid VYDAC A® oder ein äquivalentes Siliciumdioxid bedeutet.

8. Verwendung der Träger der Formel (I) bei der Synthese von Oligonucleotiden nach der Methode mit Phosphotriestern in fester Phase.

9. Neue Desoxyribonucleoside oder Ribonucleoside auf Trägern der Formel

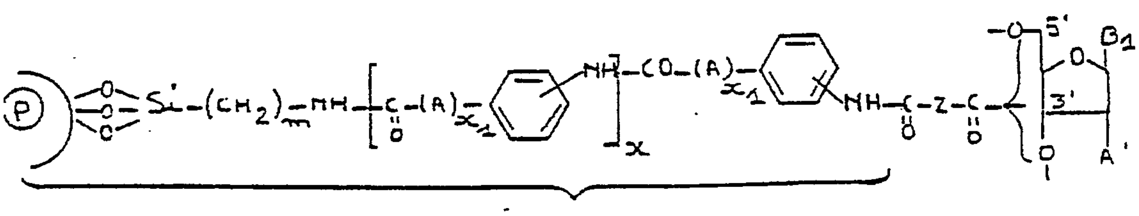

Formel (I)

worin der Träger der Formel (I) an ein Ribonucleosid oder ein Desoxyribonucleosid entweder in 3'- oder in 5'-Stellung über eine Gruppe

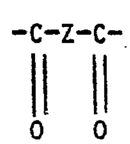

gebunden ist, wobei Z für eine Kohlenwasserstoffgruppe mit 2 bis 20 Kohlenstoffatomen oder eine Phenylgruppe steht, die gegebenenfalls geschützte Hydroxylgruppe sich entweder in 3′- oder in 5′-Stellung befindet, und worin A′ entweder für ein Wasserstoffatom steht, wenn der Träger der Formel (I) an ein Desoxyribonucleosid gebunden ist, oder für $OR_1$ steht, wenn der Träger der Foreml (I) an ein Ribonucleosid gebunden ist, wobei $R_1$ entweder ein Wasserstoffatom oder eine übliche Schutzgruppe für die Hydroxylfunktion wiedergibt, und $B_1$ eine Purin- oder Pyrimidinbase ist, deren Aminfunktion gegebenenfalls geschützt ist.

10. Neue Desoxyribonucleoside auf Trägern gemäß Anspruch 9 der Formel

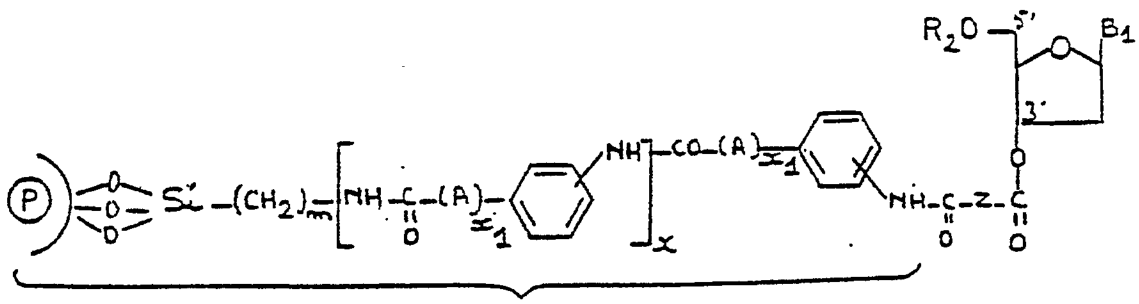

Formel (I)

worin der Träger der Formel (I) an ein Desoxyribonucleosid in 3′-Stellung über eine Gruppe

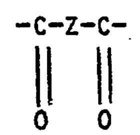

gebunden ist, worin Z für eine Kohlenwasserstoffgruppe mit 2 bis 20 Kohlenstoffatomen oder eine Phenylgruppe steht, und die Hydroxylfunktion in 5′-Stellung gegebenenfalls durch eine übliche Schutzgruppe $R_2$ geschützt ist, und $B_1$ eine Purin- oder Pyrimidinbase ist, deren Aminfunktion gegebenenfalls geschützt ist.

11. Neue Oligodesoxyribonucleotide oder Oligoribonucleotide auf Trägern, bei denen das Nucleosid entweder in 3′- oder in 5′-Stellung an den Träger der Formel (I) gebunden ist, der folgenden Formel

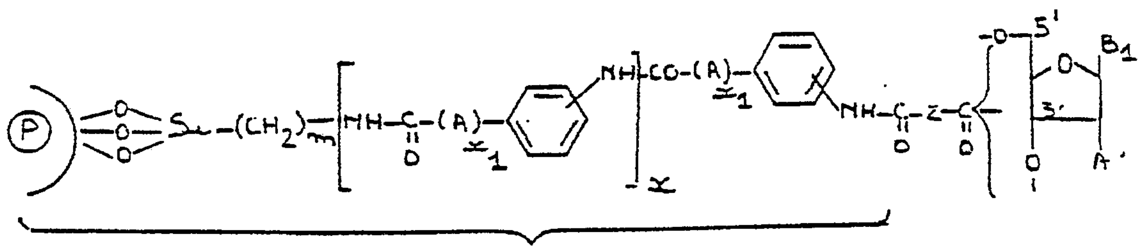

Formel (I)

und seinerseits durch Phosphodiester- oder -triester-Bindungen des Typs

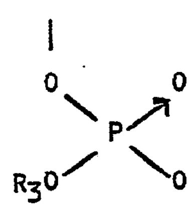

worin $R_3$ entweder ein Wasserstoffatom oder eine Schutzgruppe bedeutet, an andere Nucleotide, die Basen $B_2$, ... B(y−1) aufweisen, bis zu dem letzten Nucleosid der Formel

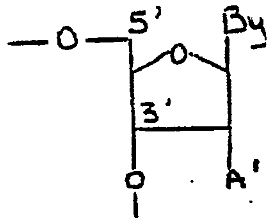

gebunden ist, worin By die letzte Base des Oligodesoxy- oder Oligoribonucleotids bedeutet, wobei die Hydroxylfunktion des letzten Nucleosids in 5'- oder in 3'-Stellung gegebenenfalls geschützt ist, und die Aminfunktionen der verschiedenen Purin- oder Pyrimidinbasen gegebenenfalls geschützt sind.

12. Neue Oligodesoxyribonucleotide auf Trägern gemäß Anspruch 11 der Formel

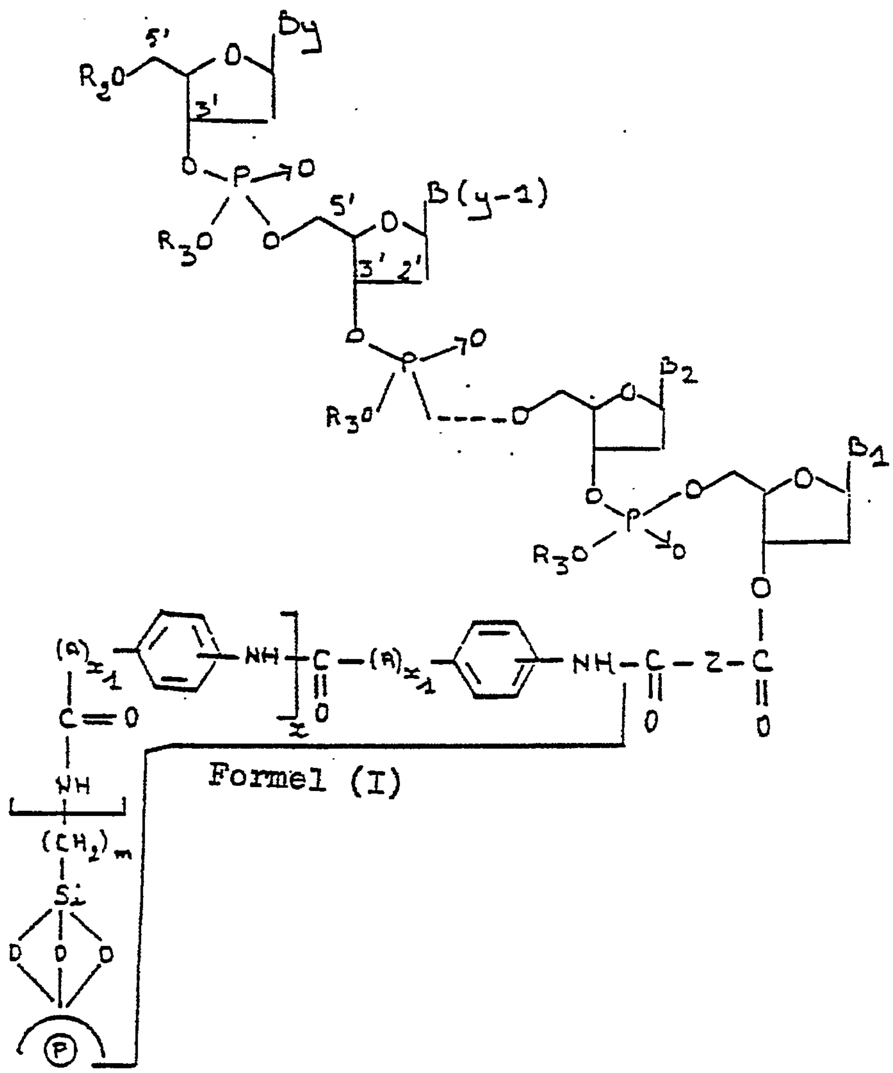

Formel (I)

worin der Träger der Formel (I) in 3'-Stellung an ein Oligodesoxyribonucleotid gebunden ist, das Basen $B_1$, $B_2$----By aufweist, und worin $R_2$ entweder ein Wasserstoffatom oder eine Schutzgruppe bedeutet, $R_3$ entweder für ein Wasserstoffatom oder eine Schutzgruppe steht und die Aminfunktionen der Purin- oder Pyrimidinbasen gegebenenfalls geschützt sind.

13. Neue Desoxyribonucleoside, Ribonucleoside, Oligodesoxyribonucleotide oder Oligoribonucleotide auf Trägern gemäß den Ansprüchen 9 bis 12, bei denen Z den Rest $-(CH_2)_2-$ wiedergibt.

## Claims

1. Use for solid phase synthesis of oligonucleotides by the method involving phosphoramidites, phosphites, phosphodiesters and phosphotriesters of the supports of formula (I):

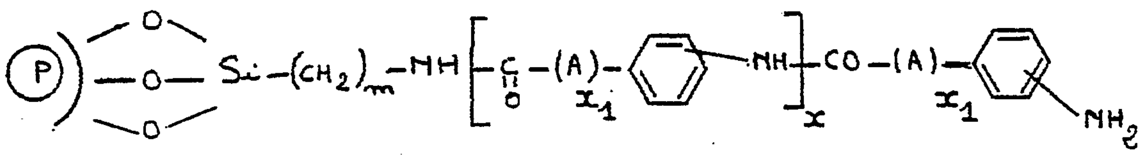

in which ® is a material constituted by glass, silica, kieselguhr, polytetrafluoroethylene, metallic oxides or cellulose microbeads

- m is an integer which can vary from 1 to 20,
- A represents either a linear or branched alkyl chain containing from 1 to 20 carbon atoms, or a saturated cyclic radical containing from 3 to 12 carbon atoms, or a phenyl radical, or a heterocyclic radical containing 5 or 6 ring members,
- x is an integer which can vary from 0 to 20,
- $x_1$ is an integer which can vary from 0 to 10, the amine functions on the phenyl nucleus can be in para, meta or ortho positions.

2. Use according to claim 1, of supports of formula (I) for which ® is a material constituted by silica having a homogeneous granulometry and for which the amine functions on the phenyl nucleus are in either meta, or para positions.

3. Use according to claims 1 or 2 of the supports of formula (I) for which m is an integer which can vary from 1 to 5.

4. Use according to claims 1 to 3 of the supports of formula (I), for which A is a $-CH_2-$ radical.

5. Use according to claim 1 to 4 of the supports of formula (I) for which x is an integer which can vary from 0 to 10 and $x_1$ is an integer which can vary from 0 to 5.

6. Use according to claims 1 to 5 of the supports of formula (I) for which m = 3.

7. Use according to claims 1 to 6 of the supports of the following formulae:

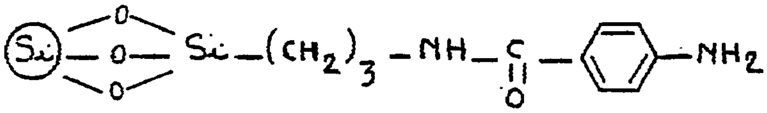

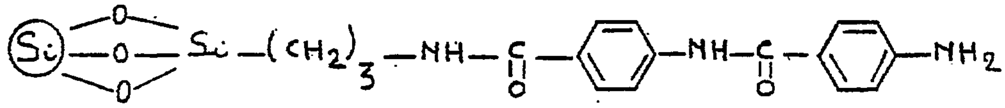

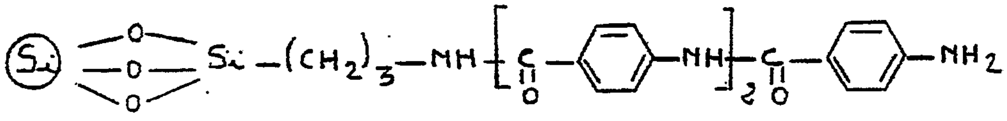

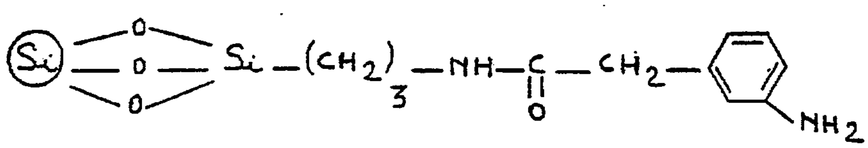

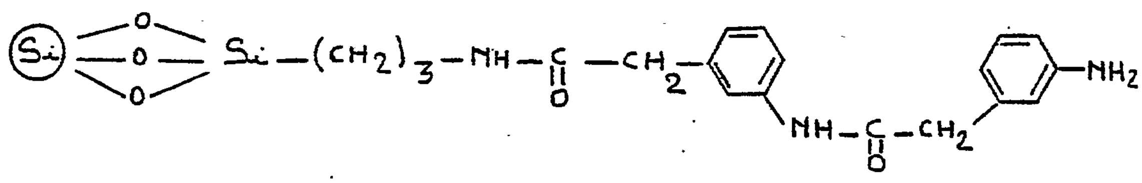

the material Si being a VYDAC A® silica or an equivalent silica.

8. Use of the supports of formula (I) for the solid phase synthesis of oligonucleotides, by the phosphotriester method.

9. New desoxyribonucleosides or ribonucleosides on supports of formula:

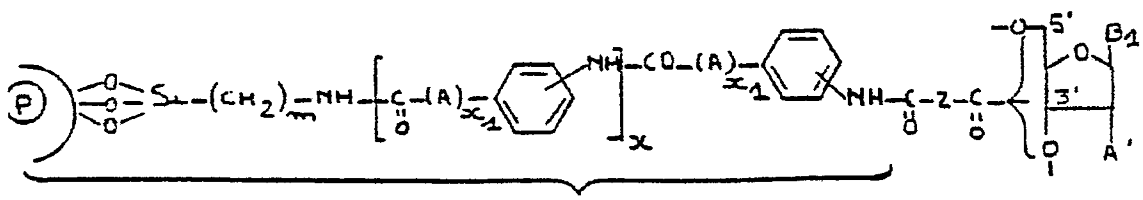

formula (I)

in which the support of formula (I) is linked to a ribonucleoside or a desoxyribonucleoside, either in position 3′, or in position 5′, by the intermediary of a

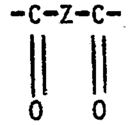

group, Z being a hydrocarbon group containing from 2 to 20 carbon atoms, or a phenyl group, the hydroxyl function being optionally protected, either in position 3′ or 5′ and in which A′ is either a hydrogen atom if the support of formula (I) is linked to a desoxyribonucleoside, or $OR_1$ if the support of formula (I) is linked to a ribonucleoside, $R_1$ being either a hydrogen atom, or a usual protector group of the hydroxyl function, $B_1$ is a purine or pyrimidine base of which the amine function is optionally protected.

10. New desoxyribonucleosides on the supports, as defined in claim 9, in formula:

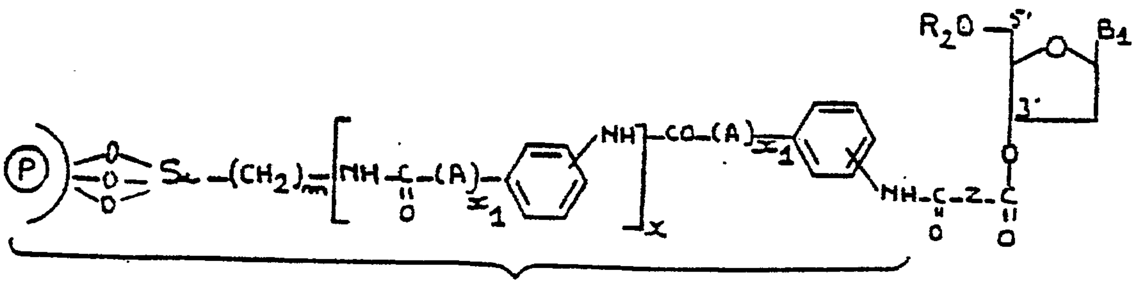

formula (I)

in which the support of formula (I) is linked to a desoxyribonucleoside in position 3′ by the intermediary of a

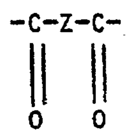

group, in which Z is a hydrocarbon group containing from 2 to 20 carbon atoms or a phenyl group and the hydroxyl function in position 5′ is optionally protected by a usual $R_2$ protector group, $B_1$ is a purine or pyrimidine base, of which the amine function is optionally protected.

11. New oligodesoxyribonucleotides or oligoribonucleotides on supports for which the nucleoside is attached either in position 3′ or in 5′, to the support of formula (I), correspond to the following formula:

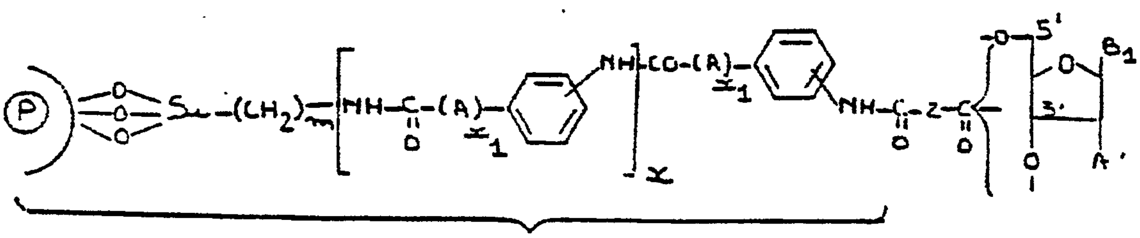

formula (I)

and is itself linked by phosphodiesters or triesters of the type:

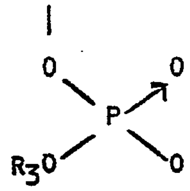

in which $R_3$ is either a hydrogen atom, or a protector group, to other nucleotides bearing the bases $B_2$, ... B(y–1) until the final nucleoside of formula:

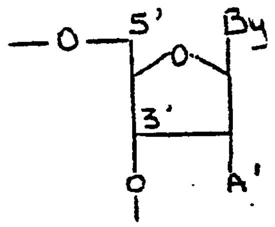

By being the final base of the oligodesoxy- or oligo-ribonucleotide, the hydroxyl function of the final nucleoside in position 5′ or 3′ being optionally protected and the different purine or pyrimidine bases having their amine functions optionally protected.

12. New oligodesoxyribonucleotides on supports, as defined in claim 11, of the formula:

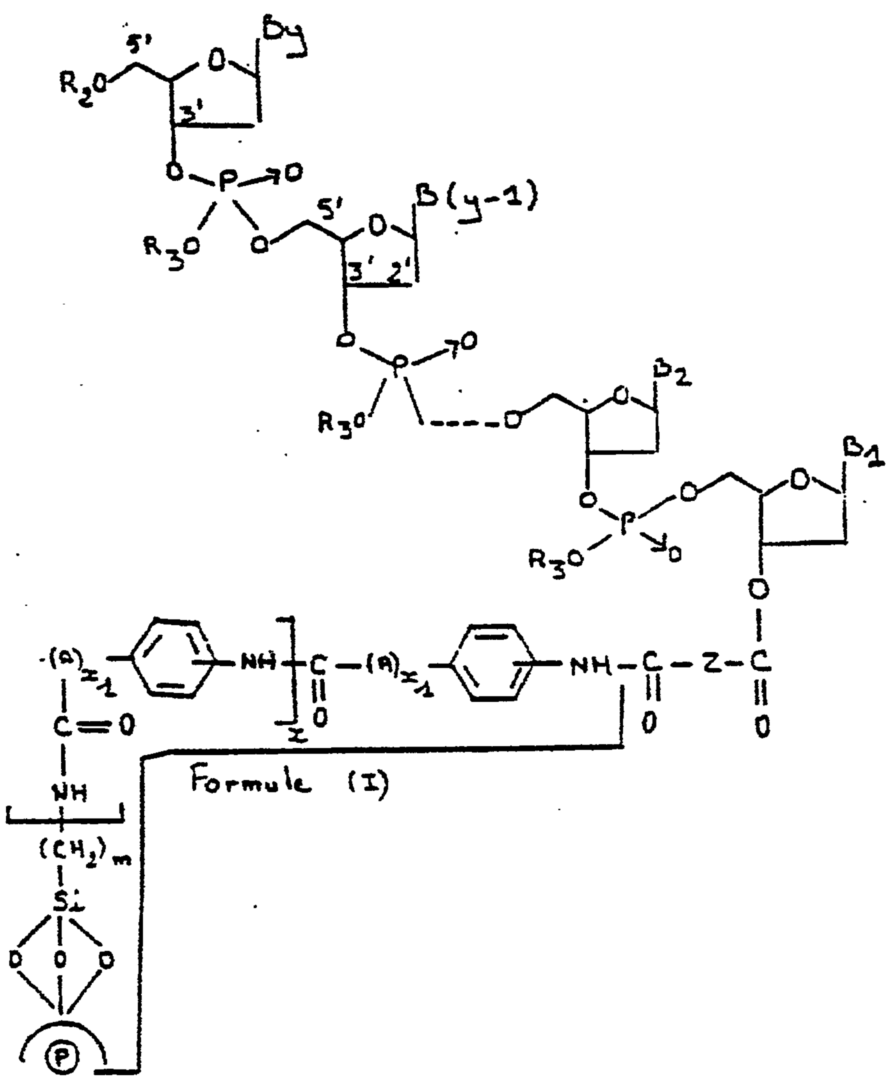

in which the support of formula (I) is linked in position 3′ to a oligodesoxyribonucleotide bearing the bases $B_1$, $B_2$ ... By and in which $R_2$ is either a hydrogen atom, or a protector group, $R_3$ is either a hydrogen atom, or a protector group, the purine or pyrimidine bases having their amine functions optionally protected.

13. New desoxyribonucleosides, ribonucleosides, oligodesoxyribonucleotides or oligoribonucleotides on supports as defined in claims 9 to 12 for which Z is the radical $-(CH_2)_2-$.